(19) Europäisches Patentamt
European Patent Office
Office européen des brevets

(11) **EP 4 509 206 B1**

(12) **EUROPÄISCHE PATENTSCHRIFT**

(45) Veröffentlichungstag und Bekanntmachung des Hinweises auf die Patenterteilung:
**12.11.2025 Patentblatt 2025/46**

(21) Anmeldenummer: **24207481.3**

(22) Anmeldetag: **05.04.2017**

(51) Internationale Patentklassifikation (IPC):
*B01D 61/14* (2006.01)    *B01D 61/16* (2006.01)
*B01D 63/08* (2006.01)    *A23C 9/142* (2006.01)
*A61M 1/34* (2006.01)    *B01D 61/22* (2006.01)
*B01D 61/32* (2006.01)

(52) Gemeinsame Patentklassifikation (CPC):
**A23C 9/142; A61M 1/3417; B01D 61/149;**
**B01D 61/16; B01D 61/22; B01D 61/32;**
**B01D 63/084;** A61M 2205/3331; B01D 2311/04;
B01D 2311/25; B01D 2311/2523; B01D 2313/143;
B01D 2315/06; B01D 2315/10; B01D 2317/022;
(Forts.)

(54) **CROSSFLOW-FILTRATION ZUR KONTINUIERLICHEN DIAFILTRATION**

CROSS-FLOW FILTRATION FOR CONTINUOUS DIAFILTRATION

FILTRATION TANGENTIELLE POUR DIAFILTRATION CONTINUE

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **05.04.2016 DE 102016004115**

(43) Veröffentlichungstag der Anmeldung:
**19.02.2025 Patentblatt 2025/08**

(62) Dokumentnummer(n) der früheren Anmeldung(en) nach Art. 76 EPÜ:
**17719481.8 / 3 439 768**

(73) Patentinhaber: **Sartorius Stedim Biotech GmbH
37079 Göttingen (DE)**

(72) Erfinder:
• **LEUTHOLD, Martin
37079 Göttingen (DE)**
• **GRUMMERT, Ulrich
37242 Bad Sooden-Allendorf (DE)**
• **SCHMIDT, Peter
37120 Bovenden (DE)**

(74) Vertreter: **Novagraaf International SA
Chemin de l'Echo 3
1213 Onex, Geneva (CH)**

(56) Entgegenhaltungen:
US-A- 4 789 468    US-A1- 2005 258 100
US-A1- 2015 096 936

(52) Gemeinsame Patentklassifikation (CPC): (Forts.)
B01D 2319/06

## Beschreibung

Die vorliegende Erfindung betrifft eine Crossflow-Filtrationseinheit zur kontinuierlichen Diafiltration einer Speiseflüssigkeit zum Erhalten eines Retentats und eines Permeats, ein entsprechendes Verfahren zur Diafiltration und die Verwendung der Crossflow-Filtrationseinheit.

Bei der Crossflow-Filtration, welche auch als Querstrom- oder Tangentialstromfiltration bezeichnet werden kann, überströmt eine zu filtrierende Speiseflüssigkeit ("Feed") tangential die Oberfläche eines Filtermaterials, welches üblicherweise eine Membran ist, und wird dabei in ein Retentat (Konzentrat) und ein Permeat (Filtrat) unterschiedlicher Zusammensetzungen aufgespalten.

Das Retentat überströmt die Oberfläche des Filtermaterials und kann nach lediglich einmaligem Überströmen entnommen werden ("Single-Pass"-Betrieb), aber auch im Kreislauf wieder zurückgeführt werden, so dass es die Membranoberfläche wiederholt überströmt. Das Permeat durchströmt die Membran senkrecht zur Oberfläche und wird danach abgeführt. Zu gewinnende Zielsubstanzen können im Permeat (Permeatstoffe) und/oder im Retentat (Retentatstoffe) enthalten sein.

Crossflow-Filtrationseinheiten werden häufig in Form von Filterkassetten eingesetzt, wie sie beispielsweise in der DE-PS 34 41 249 beschrieben sind. Filterkassetten umfassen mehrere benachbarte Crossflow-Filtrationseinheiten (Filterzellen), die in der Regel aus wiederholten Anordnungen eines Retentatspalts für die zu filtrierende Speiseflüssigkeit bzw. das Retentat, einer flächigen Membranlage und einem Permeatsammelspalt bestehen. Der Permeatsammelspalt der vorstehenden Filterzelle wird durch eine weitere flächige Membranlage von dem Retentatspalt der nächsten Filterzelle abgegrenzt. Jeder Retentatspalt ist mit einem Einlass für die zu filtrierende Speiseflüssigkeit und mit einem Auslass für das Retentat fluidleitend (kommunizierend) verbunden und jeder Permeatsammelspalt ist mit einem Auslass für das Permeat fluidleitend verbunden. US2005/285100 befasst sich mit der Diafiltration und erwähnt Proteine. Es wird der Aufbau einer Diafiltrationseinheit, mit einem Diafiltrations-, einem Feed- und einem Permeatspalt gezeigt.

Durch herkömmliche Filtration wird eine Speiseflüssigkeit in ein Retentat und ein Permeat aufgetrennt. In der Diafiltration wird diese Auftrennung mit einem Schritt des Zugebens eines Diafiltrationsmediums zu der Speiseflüssigkeit/dem Retentat kombiniert. Dadurch ist es beispielsweise möglich, eine gemeinsame Lösung von Zielsubstanz(en) und einer oder mehrerer weiterer Substanzen von den weiteren Substanzen zu befreien. Beispielsweise kann durch Diafiltration an einer Proteinlösung ein Pufferaustausch oder eine Entsalzung, gegebenenfalls kombiniert mit einer Aufkonzentration, erzielt werden.

Es wird zwischen zwei grundlegenden Arten der Diafiltration unterschieden, der volumenvariablen Diafiltration (im Stand der Technik gelegentlich als "diskontinuierliche" Diafiltration bezeichnet) und der volumenkonstanten Diafiltration (im Stand der Technik gelegentlich als "kontinuierliche" Diafiltration bezeichnet).

In der volumenvariablen Diafiltration wechseln sich Filtrationsschritte und Schritte des Zugebens eines Diafiltrationsmediums ab. Folglich schwankt im Verlauf dieser Verfahrensvariante das Volumen des Retentats, woher auch der Begriff der "diskontinuierlichen Diafiltration" rührt. In der volumenkonstanten Diafiltration wird das Volumen des Retentats dadurch konstant gehalten, dass sowohl die Filtration als auch die Zugabe des Diafiltrationsmediums kontinuierlich erfolgen. Allerdings erfolgt die Zugabe der Speiseflüssigkeit diskontinuierlich und in der Regel ausschließlich zu Beginn des Verfahrens. Daher sind sowohl die volumenvariable als auch die volumenkonstante Diafiltration diskontinuierliche Verfahren. Bei einem diskontinuierlichen Diafiltrationsverfahren muss ein festgelegtes Speiseflüssigkeitsvolumen komplett einem Verfahrensdurchlauf unterzogen werden, bevor ein neuer Verfahrensdurchlauf begonnen werden kann ("Batch"-Verfahren). Aus Gründen der Wirtschaftlichkeit und Effizienz sind kontinuierliche Verfahren, in denen laufend Stoffströme zu- und abgeführt werden können, diskontinuierlichen Verfahren vorzuziehen. Daher besteht der Bedarf an einem kontinuierlichen Diafiltrationsverfahren.

Der vorliegenden Erfindung liegt somit die Aufgabe zugrunde, eine Crossflow-Filtrationseinheit bereitzustellen, welche für die kontinuierliche Diafiltration geeignet ist.

Diese Aufgabe wird durch die in den Ansprüchen gekennzeichneten Ausführungsformen gelöst.

Insbesondere betrifft die vorliegende Erfindung eine Crossflow-Filtrationseinheit zur kontinuierlichen Diafiltration einer Speiseflüssigkeit zum Erhalten eines Retentats und eines Permeats, mindestens umfassend einen Diafiltrationspalt, ein flächiges erstes Filtermaterial, einen Retentatspalt, ein flächiges zweites Filtermaterial und einen Permeatsammelspalt, derart angeordnet, dass das flächige erste Filtermaterial den Diafiltrationspalt und den Retentatspalt voneinander abgrenzt und das flächige zweite Filtermaterial den Retentatspalt und den Permeatsammelspalt voneinander abgrenzt, wobei der Diafiltrationspalt fluidleitend mit mindestens einem Einlass für das Diafiltrationsmedium, der Retentatspalt fluidleitend mit mindestens einem Einlass für die Speiseflüssigkeit und mit mindestens einem Auslass für das Retentat und der Permeatsammelspalt fluidleitend mit mindestens einem Auslass für das Permeat verbunden ist, und wobei die Porengröße beziehungsweise die Molekulargewichtsausschlussgrenze des flächigen ersten Filtermaterials mindestens so groß wie die Porengröße beziehungsweise die Molekulargewichtsausschlussgrenze des flächigen zweiten Filtermaterials ist. Vorzugsweise ist die Porengröße beziehungsweise die Molekulargewichtsausschlussgrenze des flächigen ersten Filtermaterials größer als die Porengröße beziehungsweise die Molekulargewichtsausschlussgrenze des flächigen zweiten Filtermaterials.

Vorzugsweise weist das flächige erste Filtermaterial eine Molekulargewichtsausschlussgrenze (molecular weight cut-off, MWCO) im Bereich von 30 kDa bis 1500 kDa auf. Das flächige zweite Filtermaterial weist vorzugsweise eine Molekulargewichtsausschlussgrenze im Bereich von 5 kDa bis 1500 kDa auf. Die Bestimmung der Molekulargewichtsausschlussgrenze kann gemäß US-Standard ASTM E1343-90 ("Standard test method for molecular weight cutoff evaluation of flat sheet ultrafiltration membranes") erfolgen.

Das flächige erste Filtermaterial weist vorzugsweise eine Porengröße von 0,01 bis 50 $\mu$m, vorzugsweise 0,01 bis 0,5 $\mu$m auf. Das flächige zweite Filtermaterial weist vorzugsweise eine Porengröße von weniger als 0,01 $\mu$m auf. Die Bestimmung der Porengröße, welche auch als "größter Porendurchmesser" oder "$d_P$" und im Englischen als "maximum pore size" bezeichnet wird, kann gemäß US-Standard ASTM F316-03 TEST METHOD A ("Standard test methods for pore size characteristics of membrane filters by bubble point and mean flow pore test") erfolgen.

Grundlegende Verfahren zur Charakterisierung von Membranen sind in der Dissertationsschrift von Melanie Sossna "Strukturausbildung von Celluloseestermembranen", Universität Hannover 2006, Abschnitt 2.5 auf Seiten 10f, insbesondere in Tabelle 2-4, beschrieben.

Gemäß dem Stand der Technik (diskontinuierliche Diafiltration) wird das Diafiltrationsmedium zwischen in Reihe geschalteten Crossflow-Filtrationseinheiten eingetragen oder die Speiseflüssigkeit mit Diafiltrationsmedium verdünnt, kombiniert mit vorgelagerter oder anschließender Konzentrierung durch Crossflow-Filtration. Durch die erfindungsgemäße Crossflow-Filtrationseinheit besteht jedoch die Möglichkeit, mittels geeigneter Druckbeaufschlagung eine bestimmte Menge an Diafiltrationsmedium für eine optimale Diafiltration in den Retentatspalt einzubringen. Beispielsweise kann erfindungsgemäß das Volumen des Diafiltrationsmediums das 0,1- bis 15-fache des Volumens der Speiseflüssigkeit betragen. Vorzugsweise wird das Diafiltrationsmedium derart in den Retentatspalt eingebracht, dass die gesamte zur Verfügung stehende Fläche des flächigen ersten Filtermaterials mit dem Diafiltrationsmedium bedeckt ist. Durch die erfindungsgemäße Crossflow-Filtrationseinheit kann ein im Vergleich zur diskontinuierlichen Diafiltration verbessertes Diafiltrationsergebnis erhalten werden. Außerdem führt die Erfindung zu einer Reduzierung der Prozesszeiten und des apparativen Aufwandes.

In der kontinuierlichen Diafiltration werden sowohl Speiseflüssigkeit als auch Diafiltrationsmedium laufend zugegeben, so dass das Verfahren nicht unterbrochen werden muss. Dadurch ermöglicht die erfindungsgemäße Crossflow-Filtrationseinheit eine effiziente und wirtschaftliche Verfahrensführung.

Mit der erfindungsgemäßen, verbesserten Crossflow-Filtrationseinheit können Fluide wie Flüssigkeiten, Emulsionen, Suspensionen, Getränke, wie Bier, Wein, Saft, Wasser, Milch und Molke, Bierwürze, Brauch- und Abwasser, Lösungen im Pharma-, Medizin-, Kosmetik-, Chemie-, Biotechnologie-, Gentechnik-, Umweltschutz- und Laborbereich als Speiseflüssigkeit eingesetzt und diafiltriert werden. Sie können zur Wertstoffgewinnung, Stofftrennung beispielweise von Makromolekülen und Biomolekülen, zur Entpyrogenisierung und Sterilisierung von Lösungen, zur Abtrennung von Schadstoffen aus den Fluiden, für die (Dia)filtration und Aufkonzentrierung biologischer Lösungen, für die Abtrennung von Mikroorganismen, wie Bakterien, Hefen, Viren und von Zellbestandteilen, für die Entsalzung von Proteinlösungen und anderen biologischen Medien verwendet werden.

Besonders vorteilhaft kann die erfindungsgemäße Crossflow-Filtrationseinheit zur Filtration, Diafiltration, Aufkonzentrierung (Reduktion des Lösemittel- bzw. Wassergehaltes), und/oder Änderung der Ionenzusammensetzung (z. B. Entsalzung oder Pufferaustausch) einer Lösung, vorzugsweise einer Proteinlösung, verwendet werden.

Für die im Retentatspalt befindliche Flüssigkeit können die Begriffe Speiseflüssigkeit und Retentat gleichbedeutend verwendet werden.

Der Begriff "flächig" zeigt an, dass das jeweilige Filtermaterial im Wesentlichen in einer einzigen Ebene liegt. Vorzugsweise liegen alle Filtermaterialien im Wesentlichen in Ebenen, die weitgehend parallel zueinander sind.

Gemäß einer bevorzugten Ausführungsform der Erfindung ist das flächige erste Filtermaterial eine erste Filtrationsmembran. Das flächige zweite Filtermaterial ist vorzugsweise eine zweite Filtrationsmembran. Besonders bevorzugt ist das flächige erste Filtermaterial eine erste Filtrationsmembran und das flächige zweite Filtermaterial ist eine zweite Filtrationsmembran.

Als erstes Filtermaterial ist insbesondere eine poröse Membran im Ultrafiltrations- und Mikrofiltrationsbereich geeignet. Als zweites Filtermaterial kann vorteilhaft eine Ultrafiltrationsmembran eingesetzt werden. Durch diese Ausgestaltung kann mittels geeigneter Druckbeaufschlagung eine bestimmte Menge an Diafiltrationsmedium für eine optimale Diafiltration in den Retentatspalt eingebracht werden. Bei Druckbeaufschlagung ist die gesamte zur Verfügung stehende Fläche des flächigen ersten Filtermaterials mit dem Diafiltrationsmedium bedeckt.

Die Ultrafiltrationsmembranen sind durch Porengrößen von kleiner als 0,01 $\mu$m gekennzeichnet, beziehungsweise durch Molekulargewichtsausschlussgrenzen charakterisiert, die etwa im Molmassenbereich von 5 bis 1500 kDa liegen, während die Mikrofiltrationsmembranen Porengrößen im Bereich von 0,01 bis 50 $\mu$m, vorzugsweise 0,01 bis 0,5 $\mu$m, beziehungsweise Molekulargewichtsausschlussgrenzen von 30 bis 1500 kDa aufweisen. Die Filtrationsmembranen können beispielsweise aus Polyvinylidenfluorid, Cellulose und deren Derivaten, Polyethersulfon oder Polysulfon bestehen, wobei vernetztes Cellulosehydrat besonders bevorzugt ist.

Vorzugsweise ist der Einlass für die Speiseflüssigkeit in einem ersten Randbereich der Crossflow-Filtrationseinheit angebracht und der Auslass für das Retentat in einem zweiten Randbereich der Crossflow-Filtrationseinheit, welcher dem ersten Randbereich gegenüberliegt, angebracht. Durch diese Anordnung wird eine weitgehend einheitliche Flussrichtung des Retentats vom Einlass für die Speiseflüssigkeit als Startpunkt zum Auslass für das Retentat als Endpunkt definiert. Die Flussrichtung des Retentats erfolgt dadurch weitgehend parallel zum Strömungsweg entlang des flächigen Filtermaterials, das heißt im Wesentlichen ohne Umlenkungen, wodurch ein stabiler und zuverlässiger Fluss des Retentats durch die Crossflow-Filtrationseinheit gewährleistet werden kann. Zudem können durch den weitgehend geradlinigen Strömungsverlauf ohne Umlenkungen, Schleifen oder ähnlichem der Druckabfall in der Filtrationseinheit sowie unerwünschte Einwirkungen nicht geradliniger Strömungen auf in der Speiseflüssigkeit enthaltene Zielsubstanzen minimiert werden. Aus den vorstehenden Gründen ist zudem bevorzugt, dass der Einlass für das Diafiltrationsmedium im ersten Randbereich der Crossflow-Filtrationseinheit angebracht ist. Es ist allerdings auch möglich, den Einlass für das Diafiltrationsmedium im zweiten Randbereich anzubringen, beziehungsweise im dritten und/oder vierten Randbereich.

Gemäß einer bevorzugten Ausgestaltung der Erfindung ist der Auslass für das Permeat im zweiten Randbereich der Crossflow-Filtrationseinheit angebracht. Besonders bevorzugt ist sowohl im ersten als auch im zweiten Randbereich der Crossflow-Filtrationseinheit jeweils mindestens ein Auslass für das Permeat angebracht. In einer weiteren Ausgestaltung der Erfindung sind die Auslässe für das Permeat alternativ oder ergänzend im dritten und/oder vierten Randbereich der Crossflow-Filtrationseinheit angebracht. Der dritte Randbereich befindet sich bei einer Draufsicht auf die Crossflow-Filtrationseinheit von der Seite des Diafiltrationsspaltes auf der linken Seite der Flussrichtung. Der vierte Randbereich befindet sich entsprechend auf der rechten Seite und liegt somit dem dritten Randbereich gegenüber. Durch vorstehende Anordnung des Auslasses oder der Auslässe können eine besonders hohe Permeatleistung erzielt und konstruktionstechnische Vorteile erreicht werden.

Vorzugsweise umfasst der erste Randbereich das äußere Drittel der Länge der Filtrationseinheit entgegen der Flussrichtung. Entsprechend umfasst der zweite Randbereich das äußere Drittel der Länge der Filtrationseinheit entlang der Flussrichtung. Entsprechendes gilt für die dritten und vierten Randbereiche. Es ist von Vorteil, die ersten bis vierten Randbereiche so klein wie möglich zu gestalten. Daher umfassen die Randbereiche besonders bevorzugt die jeweiligen äußeren 20%, noch mehr bevorzugt die jeweiligen äußeren 10% und am meisten bevorzugt die jeweiligen äußeren 3%.

Grundsätzlich besteht hinsichtlich der Anbringung der Ein- und Auslässe keine besondere Einschränkung. Zum Beispiel können die Ein- und Auslässe so angebracht sein, dass die Speiseflüssigkeit bereits in Flussrichtung in den Retentatspalt eintritt und ihn in Flussrichtung verlässt. Entsprechend kann der Auslass für das Permeat so angebracht sein, dass das Permeat den Permeatsammelspalt in Flussrichtung verlässt und/oder der Einlass für das Diafiltrationsmedium kann so angebracht sein, dass es in Flussrichtung in den Diafiltrationsspalt eintritt. Vorzugsweise sind die Ein- und Auslässe jedoch so angebracht, dass das Diafiltrationsmedium senkrecht zur Flussrichtung in den Diafiltrationsspalt eintritt und die Speiseflüssigkeit zunächst senkrecht zur Flussrichtung in den Retentatspalt eintritt und diesen senkrecht zur Flussrichtung als Retentat verlässt. Eine derartige Anbringung der Ein- und Auslässe erleichtert die Anordnung einer Mehrzahl der erfindungsgemäßen Filtrationseinheiten zu einer Filterkassette.

Vorzugsweise weist die Crossflow-Filtrationseinheit mehrere Einlässe für die Speiseflüssigkeit, mehrere Auslässe für das Retentat und mehrere Auslässe für das Permeat auf.

In einer bevorzugten Ausgestaltung nimmt das freie Volumen des Diafiltrationsspaltes und/oder des Retentatspaltes (für das Diafiltrationsmedium/Retentat zur Verfügung stehender Raum, Tot- oder Leervolumen) in der Flussrichtung vom Einlass für die Speiseflüssigkeit zum Auslass für das Retentat ab. Durch das/die abnehmenden Volumen/Volumina und die Flächigkeit der Filtermaterialien weist die Crossflow-Filtrationseinheit einen niedrigen Druckverlust und einen im Wesentlichen umlenkungsfreien Strömungsweg des Diafiltrationsmediums und des Retentats auf. Dadurch ist es möglich, die Flächenleistung der Crossflow-Diafiltrationseinheit zu erhöhen und die Crossflow-Diafiltrationseinheit im "Single-Pass"-Betrieb zu betreiben (es findet nur ein einziger Durchgang des Retentats ohne Kreislaufführung statt).

In einer weiteren bevorzugten Ausgestaltung der Erfindung wird die Abnahme des freien Volumens entlang der Flussrichtung dadurch realisiert, dass die Breite des Diafiltrationsspaltes und/oder die Breite des Retentatspaltes in Flussrichtung abnimmt. Die Breite verläuft entlang des flächigen ersten Filtermaterials und senkrecht zur Flussrichtung. Besonders bevorzugt nimmt die Breite der gesamten Crossflow-Filtrationseinheit in Flussrichtung ab. Vorzugsweise ist der Retentatspalt bzw. die Crossflow-Filtrationseinheit in einer Draufsicht entlang einer Normalen der Ebene, in der das flächige erste Filtermaterial liegt, trapezförmig. Die Trapez-Grundform des Diafiltrationsspaltes und/oder des Retentatspaltes bzw. der Crossflow-Filtrationseinheit kann ungleichschenklig, beispielsweise rechtwinklig, sein und ist vorzugsweise gleichschenklig.

Gemäß einer Ausführungsform kann die Höhe des Diafiltrationsspaltes und/oder des Retentatspaltes bzw. der Crossflow-Filtrationseinheit in Flussrichtung abnehmen. Beispielsweise können der Diafiltrationsspalt und/oder der Retentatspalt keilförmig ausgestaltet sein. Die Höhe des Diafiltrationsspaltes und/oder des Retentatspaltes bzw. der Crossflow-Filtrationseinheit verläuft senkrecht zum flächigen ersten Filtermaterial und senkrecht zur Flussrichtung.

Hinsichtlich der Breite, Länge und Höhe der Crossflow-Filtrationseinheit besteht keine besondere Einschränkung. Die Länge verläuft parallel zur Flussrichtung und entlang des flächigen ersten Filtermaterials. Vorzugsweise weist die Crossflow-Filtrationseinheit eine Länge von mindestens 50 mm, bevorzugt mindestens 150 mm, besonders bevorzugt 500 mm, insbesondere bevorzugt 750 mm oder mehr, auf. Eine derartige Länge kann beispielsweise durch Hintereinanderschalten mehrerer, beispielsweise mindestens 2, mindestens 3 oder mindestens 4, Crossflow-Filtrationseinheiten erzielt werden. Durch eine höhere Länge kann eine besonders hohe Effizienz erzielt werden.

In einer bevorzugten Ausführungsform der Erfindung ändert sich das freie Volumen des Permeatsammelspaltes in der Flussrichtung. Besonders bevorzugt nimmt das freie Volumen des Permeatsammelspaltes in der Flussrichtung ab. Dadurch können beispielsweise die äußeren Dimensionen der Filtrationskassette beibehalten werden.

Die Erläuterungen zur Ausgestaltung des Retentatspaltes gelten entsprechend für den Permeatsammelspalt und umgekehrt.

"In Flussrichtung abnehmendes freies Volumen" bedeutet, dass eine Querschnittsfläche $A_1$, welche von dem Diafiltrationsmedium beziehungsweise dem Retentat durchströmt werden kann und die in einer Ebene liegt, welche eine parallel zur Flussrichtung verlaufende Normale aufweist, und eine entsprechende Querschnittsfläche $A_2$, die parallel zu $A_1$ ist und weiter vom Einlass für das Diafiltrationsmedium beziehungsweise die Speiseflüssigkeit entfernt ist als $A_1$, wobei der von dem Diafiltrationsmedium beziehungsweise dem Retentat durchströmbare Flächeninhalt $A_1$ größer ist als $A_2$, existieren und keine entsprechend definierten Ebenen $A_1'$ und $A_2'$ existieren, für die der Flächeninhalt von $A_1'$ kleiner als der von $A_2'$ ist.

Die Abnahme des freien Volumens kann stetig (für alle $A_1$ und $A_2$ gilt $A_1 \geq A_2$) oder kontinuierlich (für alle $A_1$ und $A_2$ gilt $A_1 > A_2$) sein. Es ist auch möglich, dass die Volumenabnahme diskontinuierlich ist, das heißt, dass entlang der Flussrichtung mindestens ein unstetiger Abfall oder Sprung in der Querschnittsfläche auftritt.

Die Änderung des freien Volumens des Retentatspaltes in Flussrichtung liegt vorzugsweise im Bereich von 20 zu 1 bis 1,2 zu 1, bevorzugt bei 10 zu 1, abhängig von der Filtrationsaufgabe. Dabei wird unter der "Änderung des freien Volumens des Retentatspaltes in Flussrichtung" das Verhältnis der Querschnittsfläche $A_1$ am Einlass für die Speiseflüssigkeit zur Querschnittsfläche $A_2$ am Auslass für das Retentat verstanden.

In einer Ausgestaltung der Erfindung nimmt/nehmen die Dicke des Diafiltrationsspaltes und/oder die Dicke des Retentatspaltes und gegebenenfalls die Dicke des Permeatsammelspaltes in Flussrichtung ab.

Der Diafiltrationsspalt, der Retentatspalt und der Permeatsammelspalt werden üblicherweise durch Abstandshalter für die entsprechenden Medien offengehalten. In einer bevorzugten Ausgestaltung der Erfindung ist/sind im Diafiltrationsspalt und/oder im Retentatspalt der Crossflow-Filtrationseinheit ein flächiger Abstandshalter angebracht, derart, dass das freie Volumen des Retentatspaltes in Flussrichtung abnimmt.

Geeignete Abstandshalter für Crossflow-Filtrationseinheiten sind im Stand der Technik bekannt und können im Diafiltrationsspalt, Retentatspalt und/oder Permeatsammelspalt der erfindungsgemäßen Crossflow-Filtrationseinheit verwendet werden. Vorzugsweise sind die Abstandshalter erfindungsgemäß so modifiziert, dass ihr Volumen in Flussrichtung zunimmt, um eine Volumenabnahme des für das Diafiltrationsmedium bzw. das Retentat zur Verfügung stehenden freien Volumens zu erzielen. Bevorzugte Abstandshalter können textile Materialien aus organischen oder nicht-organischen Werkstoffen sein, wie Gewebe, Gewirke, Vliese oder extrudierte Netze.

Vorteilhaft kann der Abstandshalter eine nicht ebene Platte sein. Die nicht ebene Platte kann eine Platte sein, die zumindest eine nicht ebene Hauptoberfläche aufweist. Die Hauptoberflächen einer Platte sind die sich gegenüberstehenden Oberflächen mit dem größten Flächeninhalt. Die zumindest eine nicht ebene Hauptoberfläche kann Unebenheiten in Form einer gewellten oder gezackten Oberfläche aufweisen. Daneben kann die unebene Oberfläche vorstehende Elemente wie Kegel(stümpfe), Pyramiden(stümpfe), Noppen oder andere geometrische Figuren aufweisen. Die nicht ebene Platte kann auch ähnlich einem Wellblech in gewellter oder gezackter Form vorliegen, wobei sich die Wellen oder Zacken vorzugsweise parallel zur Flussrichtung erstrecken. Geeignete Materialien für die nicht ebene Platte sind die gleichen wie nachstehend für Abstandshalter in Form einer offenmaschigen Matrix aufgeführt.

Gemäß einer bevorzugten Ausführungsform besteht der Abstandshalter aus einer offenmaschigen Matrix oder aus einem extrudierten Netz. Derartige Abstandshalter sind im Stand der Technik bekannt und wurden beispielsweise in der Veröffentlichung der deutschen Patentanmeldung DE 100 22 259 A1 beschrieben. Wie bereits vorstehend erwähnt, sind die Abstandshalter erfindungsgemäß vorzugsweise so abgewandelt, dass ihr Volumen in Flussrichtung zunimmt, um eine Volumenabnahme des für das Diafiltrationsmedium bzw. das Retentat zur Verfügung stehenden freien Volumens zu erzielen. Grundsätzlich können in der erfindungsgemäßen Crossflow-Filtrationseinheit auch gewöhnliche Abstandshalter verbaut werden, beispielsweise im Permeatsammelspalt und/oder im Diafiltrationsspalt oder in allen Spalten der Filtrationseinheit mit in Flussrichtung abnehmender Breite. Eine abnehmende Breite kann, wie vorstehend beschrieben, beispielsweise durch eine trapezförmige Ausgestaltung der Spalten beziehungsweise der Crossflow-Filtrationseinheit erzielt werden.

In einer Ausgestaltung kann die Maschenweite der offenmaschigen Matrix beziehungsweise des extrudierten Netzes in Flussrichtung abnehmen, um eine Abnahme des freien Volumens entlang der Flussrichtung zu erzielen. Beispielsweise beträgt die Maschenzahl am Einlass für das Diafiltrationsmedium beziehungsweise am Einlass für die Speiseflüssigkeit 5/cm bis 15/cm, in der Mitte zwischen dem Einlass für das Diafiltrationsmedium beziehungsweise dem Einlass für die Speiseflüssigkeit und dem Auslass für das Retentat 10/cm bis 30/cm und am Auslass für das Retentat 20/cm bis 40/cm.

Alternativ oder ergänzend dazu kann die offenmaschige Matrix beziehungsweise das extrudierte Netz aus sich kreuzenden Längs- und Querfäden aufgebaut sein und die Anzahl und/oder Dicke der Längs- und/oder Querfäden in Flussrichtung zunehmen. Die offenmaschige Matrix besteht vorzugsweise aus einem organischen Polymer wie beispielsweise Polypropylen, Polyethylen, Polyester, Polyvinylchlorid oder Polyvinylidenfluorid oder Blends daraus. Ferner ist möglich, dass die offenmaschige Matrix aus Fasern verschiedenartiger Polymere aufgebaut ist.

In einer weiteren bevorzugten Ausgestaltung der Erfindung sind im Retentatspalt mehrere Lagen textiler Materialien derart übereinander angeordnet, dass das freie Kanalvolumen in Flussrichtung abnimmt. Dies kann beispielsweise dadurch erreicht werden, dass die übereinander angeordneten Lagen in Flussrichtung versetzt beginnen. Die übereinander angeordneten Lagen erstrecken sich vorzugsweise bis zum zweiten Randbereich. Dadurch wird im Retentatspalt in Flussrichtung ein zunehmendes Volumen von den textilen Materialien in Anspruch genommen, sodass das freie Volumen in Flussrichtung abnimmt. Die textilen Materialien, wie Gewebe, Gewirke, Vliese oder extrudierte Netze, können aus organischen oder nicht-organischen Werkstoffen bestehen.

Die hier dargestellten Ausgestaltungen zur Realisierung der Abnahme des freien Volumens des Diafiltrationsspaltes bzw. Retentatspaltes sind beliebig miteinander kombinierbar.

Erfindungsgemäß wird der Retentatspalt von einem flächigen ersten Filtermaterial und einem flächigen zweiten Filtermaterial begrenzt. Der Diafiltrationsspalt wird durch mindestens ein flächiges erstes Filtermaterial begrenzt. Der Permeatsammelspalt wird durch mindestens ein flächiges zweites Filtermaterial begrenzt. Einem Retentatspalt ist ein Diafiltrationsspalt und ein Permeatsammelspalt benachbart. Eine bevorzugte erfindungsgemäße Crossflow-Filtrationseinheit besteht aus einer Mehrzahl übereinandergestapelter Anordnungen von Diafiltrationspalt, flächigem erstem Filtermaterial, Retentatspalt, flächigem zweitem Filtermaterial, Permeatsammelspalt, flächigem zweitem Filtermaterial, Retentatspalt und flächigem erstem Filtermaterial, vorzugsweise abgeschlossen von einem weiteren Diafiltrationsspalt, sodass die gestapelten Anordnungen zu einer Filterkassette zusammengefasst sind. Geeignete Ausgestaltungen für Filterkassetten sind im Stand der Technik bekannt. Vorzugsweise ist jeder Diafiltrationsspalt dieser Anordnungen an beiden Seiten durch jeweils ein Filtermaterial, welches dem ersten Filtermaterial entspricht, von zwei Retentatspalten abgegrenzt. Entsprechend ist vorzugsweise jeder Permeatsammelspalt dieser Anordnungen an beiden Seiten durch jeweils ein Filtermaterial, welches dem zweiten Filtermaterial entspricht, von zwei Retentatspalten abgegrenzt. Die ersten Filtermaterialien und zweiten Filtermaterialien können jeweils voneinander verschieden sein. Das heißt, es können grundsätzlich verschiedenartige erste Filtermaterialien und verschiedenartige zweite Filtermaterialien verwendet werden. Vorzugsweise werden gleichartige erste Filtermaterialien und/oder gleichartige zweite Filtermaterialien verwendet.

Gemäß einer bevorzugten Ausführungsform weisen die flächigen ersten und zweiten Filtermaterialien unabhängig voneinander eine jeweils weitgehend einheitliche Dicke von vorzugsweise 50 $\mu$m bis 10000 $\mu$m, insbesondere bevorzugt 150 $\mu$m bis 1000 $\mu$m, auf. Sofern die flächige Begrenzung des Diafiltrationsspaltes durch das flächige erste Filtermaterial und die weitere flächige Begrenzung des Diafiltrationsspaltes und/oder die flächigen Begrenzungen des Retentatspaltes durch das erste und zweite Filtermaterial nicht parallel zueinander verlaufen, kann das freie Volumen des Diafiltrationsspaltes und/oder des Retentatspaltes keilförmig ausgestaltet werden, so dass in Flussrichtung das freie Volumen abnimmt. Besonders bevorzugt sind der Diafiltrationsspalt, der Retentatspalt und der Permeatsammelspalt beidseitig von weitgehend parallelen Flächen begrenzt.

Die Form der Crossflow-Filtrationseinheit unterliegt keiner besonderen Einschränkung. Die Crossflow-Filtrationseinheit kann beispielsweise quaderförmig oder zylinderförmig sein.

In einem weiteren Aspekt betrifft die vorliegende Erfindung ein Verfahren zur Diafiltration einer Speiseflüssigkeit zum Erhalten eines Retentats und eines Permeats, umfassend die Schritte

(A) Bereitstellen einer erfindungsgemäßem Crossflow-Filtrationseinheit;
(B) Zuführen eines Diafiltrationsmediums in den Einlass für das Diafiltrationsmedium;
(C) Zuführen der Speiseflüssigkeit in den Einlass für die Speiseflüssigkeit;
(D) Abführen des Retentats aus dem Auslass für das Retentat; und
(E) Abführen des Permeats aus dem Auslass für das Permeat.

Die Erläuterungen in Bezug auf die Crossflow-Filtrationseinheit und das Diafiltrationsverfahren sind wechselseitig aufeinander anwendbar.

In Schritt (A) wird bevorzugt die vorstehend beschriebene Crossflow-Filtrationseinheit bereitgestellt, welche eine Mehrzahl übereinandergestapelter Anordnungen von Diafiltrationsspalt, flächigem ersten Filtermaterial, Retentatspalt, flächigem zweiten Filtermaterial, Permeatsammelspalt, flächigem zweiten Filtermaterial, Retentatspalt und flächigem ersten Filtermaterial aufweist, sodass die gestapelten Anordnungen zu einer Filterkassette zusammengefasst sind.

Das verwendete Diafiltrationsmedium unterliegt keiner besonderen Einschränkung. Geeignet ist grundsätzlich jedes Fluid, wobei Wasser und wässrige Salzlösungen bevorzugt sind. Besonders bevorzugt ist als Diafiltrationsmedium eine wässrige Pufferlösung.

Vorzugsweise beträgt der Volumenstrom des zugeführten Diafiltrationsmediums das 0,1- bis 15-fache des Volumenstroms der zugeführten Speiseflüssigkeit. Der Volumenstrom des abgeführten Retentats beträgt vorzugsweise das 0,05- bis 10-fache des Volumenstroms der zugeführten Speiseflüssigkeit.

In einer bevorzugten Ausgestaltung des Verfahrens wird das Diafiltrationsmedium mit einem Druck von 0,1 bis 4 bar zugeführt. Besonders bevorzugt wird das Diafiltrationsmedium mit einem Druck zugeführt, der größer ist als der Retentatausgangsdruck.

Vorzugsweise wird das erfindungsgemäße Verfahren kontinuierlich betrieben, das heißt unter konstanter/kontinuierlicher Zugabe des Diafiltrationsmediums und der Speiseflüssigkeit, wodurch ein besonders effizientes und wirtschaftliches Filtrationsverfahren bereitgestellt werden kann. Erfindungsgemäß wird unter "kontinuierlicher Diafiltration" ein Diafiltrationsverfahren verstanden, in dem sowohl das Diafiltrationsmedium als auch die Speiseflüssigkeit kontinuierlich zugegeben werden.

In einer bevorzugten Ausgestaltung des Verfahrens werden in Schritt (A) mehrere erfindungsgemäße und voneinander unabhängige Crossflow-Filtrationseinheiten bereitgestellt und so in Reihe geschaltet, dass der Auslass für das Retentat der jeweiligen vorgeschalteten Crossflow-Filtrationseinheit fluidleitend mit dem Einlass für die Speiseflüssigkeit einer nachgeschalteten Crossflow-Filtrationseinheit verbunden ist. In dieser Ausgestaltung wird zudem in Schritt (C) die Speiseflüssigkeit in den Einlass für die Speiseflüssigkeit derjenigen Crossflow-Filtrationseinheit, welcher keine andere Crossflow-Filtrationseinheit vorgeschaltet ist (erste Crossflow-Filtrationseinheit), zugeführt und in Schritt (D) das Retentat aus dem Auslass für das Retentat derjenigen Crossflow-Filtrationseinheit, welcher keine andere Crossflow-Filtrationseinheit nachgeschaltet ist (letzte Crossflow-Filtrationseinheit), abgeführt. Somit durchläuft das Retentat/die Speiseflüssigkeit die in Reihe geschalteten Crossflow-Filtrationseinheiten von der ersten bis zur letzten. Vorzugsweise werden 2 bis 10, besonders bevorzugt 2 bis 5 Crossflow-Filtrationseinheiten in Reihe geschaltet. Das Diafiltrationsmedium wird in dieser Ausgestaltung jeder der in Reihe geschalteten Crossflow-Filtrationseinheiten getrennt zugeführt. Es ist zwar möglich, verschiedenartige Diafiltrationsmedien zu verwenden, wobei bevorzugt ist, jeder der Crossflow-Filtrationseinheiten das gleiche Diafiltrationsmedium zuzuführen. Vorzugsweise liegt jede der in Reihe geschalteten Crossflow-Filtrationseinheiten, wie vorstehend beschrieben, in Form einer Filterkassette vor.

In einer bevorzugten Ausgestaltung des Verfahrens werden in Schritt (A) mehrere erfindungsgemäße und voneinander unabhängige Crossflow-Filtrationseinheiten bereitgestellt und parallelgeschaltet. Parallel- und Reihenschaltung können miteinander kombiniert werden.

In einer weiteren bevorzugten Ausführungsform des Verfahrens wird das in Schritt (D) abgeführte Retentat zumindest teilweise in den Einlass für die Speiseflüssigkeit zurückgeführt. Durch einen Kreislaufprozess kann, sofern ein einziger Durchlauf durch die Crossflow-Filtrationseinheit nicht ausreicht, ein verbessertes Ergebnis des Verfahrens erzielt werden. Die Zugabe der Diafiltrationsflüssigkeit und die Entnahme des Permeats erfolgen hierbei ohne Rückführung. Sind mehrere Crossflow-Filtrationseinheiten in Serie geschaltet, kann grundsätzlich jeder Retentatstrom zu jedem Einlass für die Speiseflüssigkeit zurückgeführt werden. Vorzugsweise wird hierbei das Retentat jeder einzelnen Crossflow-Filtrationseinheit zum Einlass für die Speiseflüssigkeit derselben Crossflow-Filtrationseinheit zurückgeführt.

Gemäß einer weiteren bevorzugten Ausführungsform des erfindungsgemäßen Verfahrens wird/werden die Speiseflüssigkeit beziehungsweise das Retentat im Retentatspalt in Oszillation versetzt. Dadurch wird die Strömungsbewegung im Retentatspalt durch eine Oszillation (Schwingung) überlagert. Bewerkstelligt werden kann dies durch mindestens eine Vorrichtung zur Erzeugung von Oszillation, die am Speiseflüssigkeitseinlass und/oder am Retentatauslass angebracht sein kann. Eine derartige Vorrichtung zur Erzeugung von Oszillation bewegt das Retentat im Retentatspalt hin und her, das heißt, das Retentat wird in eine zum flächigen ersten Filtermaterial weitgehend parallel verlaufende oszillierende Bewegung versetzt. Vorzugsweise wird die Oszillation von einer Vorrichtung zur Erzeugung von Oszillation, die am Speiseflüssigkeitseinlass angebracht ist, sowie einer weiteren Vorrichtung zur Erzeugung von Oszillation, die am Retentatauslass angebracht ist, erzeugt. Eine erfindungsgemäß geeignete Vorrichtung zur Erzeugung von Oszillation ist beispielsweise eine Kolbenpumpe. Bevorzugt umfasst eine Vorrichtung zur Erzeugung von Oszillation ein Reservoir, das durch eine elastische Membran in zwei Hälften unterteilt ist, und gegebenenfalls eine Druckquelle. Das Reservoir ist ein Reservoir (Speicher) für die Speiseflüssigkeit beziehungsweise das Retentat. Die erste Hälfte des Reservoirs ist über eine Ventilsteuerung mit einer Druckquelle, wie z. B. einer Druckluftquelle oder einer Pumpe, verbunden. Die zweite Hälfte des Reservoirs ist fluidleitend mit dem Speiseflüssigkeitseinlass bzw. dem Retentatauslass der Filtrationsvorrichtung verbunden. In der zweiten Hälfte befindet sich außerdem vorzugsweise ein Entleerungs- bzw. Entlüftungsventil. Durch gegenläufiges (alternierendes bzw. abwechselndes) Beaufschlagen jeweils einer der ersten Hälften der beiden Reservoire mit Druck (beispielsweise mit Druckluft) kann so die Retentatströmung hin- und herbewegt (in Oszillation bzw. Schwingung versetzt) werden. Die vorstehend beschriebenen Reservoire können sowohl separater Anlagenteil (nicht Teil der Crossflow-Filtrationseinheit) sein, als auch als Teil der Crossflow-Filtrationseinheit in deren Gehäuse integriert sein.

Vorzugsweise weist die erfindungsgemäße Crossflow-Filtrationseinheit mindestens eine (vorzugsweise zwei) Vorrichtung(en) zur Erzeugung einer Oszillation der Speiseflüssigkeit beziehungsweise des Retentats im Retentatspalt auf. Es ist erfindungsgemäß bevorzugt, dass eine Vorrichtung zur Erzeugung von Oszillation ein Reservoir mit einer Druckquelle aufweist. Besonders bevorzugt umfasst die erfindungsgemäße Crossflow-Filtrationseinheit ein erstes Reservoir, gegebenenfalls mit einer ersten Druckquelle, und ein zweites Reservoir, gegebenenfalls mit einer zweiten Druckquelle, wobei das erste Reservoir fluidleitend mit dem Einlass für die Speiseflüssigkeit verbunden ist und das zweite Reservoir fluidleitend mit dem Auslass für das Retentat verbunden ist, mit der Maßgabe, dass zumindest eine der ersten und zweiten Druckquellen vorhanden ist. Vorzugsweise weisen sowohl das erste als auch das zweite Reservoir jeweils eine Druckquelle auf. Jedes der Reservoire ist vorzugsweise durch eine elastische und fluidundurchlässige (gas- und flüssigkeitsundurchlässige) Membran in zwei Hälften unterteilt, wobei die erste Hälfte mit einer Druckquelle verbunden ist. Die zweite Hälfte des ersten Reservoirs ist vorzugsweise fluidleitend mit dem Einlass für die Speiseflüssigkeit verbunden. Die zweite Hälfte des zweiten Reservoirs ist vorzugsweise fluidleitend mit dem Auslass für das Rententat verbunden.

Gemäß einer bevorzugten Ausführungsform umfasst das erfindungsgemäße Verfahren weiter den Schritt (C0) der Auftrennung einer Vorspeiseflüssigkeit in ein Vorretentat und ein Vorpermeat. Durch diesen vorgeschalteten Schritt kann eine Flüssigkeit zunächst beispielsweise durch Filtration oder Diafiltration aufkonzentriert und/oder von Verunreinigungen (teil)befreit werden, um dann einer nachfolgenden Diafiltration unter Verwendung der erfindungsgemäßen Crossflow-Filtrationseinheit unterzogen zu werden. Sofern Schritt (C0) ein Filtrationsschritt oder Diafiltrationsschritt ist, können grundsätzlich sowohl das Vorretentat als auch das Vorpermeat als Speiseflüssigkeit verwendet werden, bevorzugt wird jedoch das Vorretentat aus Schritt (C0) verwendet.

Schritt (C0) wird vorteilhaft unter Verwendung einer Crossflow-Filtrationseinheit (C0-Einheit) zur Trennung einer Vorspeiseflüssigkeit in ein Vorretentat und ein Vorpermeat durchgeführt. Derartige Crossflow-Filtrationseinheiten sind im Stand der Technik bekannt. Die CO-Einheit kann dabei, wie vorstehend für die Reihenschaltung der erfindungsgemäßen Crossflow-Filtrationseinheiten beschrieben, der ersten Crossflow-Filtrationseinheit vorgeschaltet werden.

Besonders vorteilhaft ist als CO-Einheit eine Crossflow-Filtrationseinheit, umfassend einen Vorretentatspalt, ein flächiges Filtermaterial und einen Vorpermeatsammelspalt, derart angeordnet, dass das flächige Filtermaterial den Vorretentatspalt und den Vorpermeatsammelspalt voneinander abgrenzt, wobei der Vorretentatspalt fluidleitend mit mindestens einem Einlass für die Vorspeiseflüssigkeit und mit mindestens einem Auslass für das Vorretentat verbunden ist und der Vorpermeatsammelspalt fluidleitend mit mindestens einem Auslass für das Vorpermeat verbunden ist, der Einlass für die Vorspeiseflüssigkeit in einem ersten Randbereich der Crossflow-Filtrationseinheit angebracht ist und der Auslass für das Vorretentat in einem zweiten Randbereich der Crossflow-Filtrationseinheit, welcher dem ersten Randbereich gegenüberliegt, angebracht ist, und vorzugsweise das freie Volumen des Vorretentatspaltes in der Flussrichtung vom Einlass für die Vorspeiseflüssigkeit zum Auslass für das Vorretentat abnimmt. Durch die Abnahme des freien Volumens in Flussrichtung kann eine CO-Einheit mit geringem Druckverlust und hoher Flächenausnutzung bereitgestellt werden.

Für die Ausgestaltung der CO-Einheit und insbesondere des Vorretentatspaltes und des Vorpermeatsammelspaltes gelten die Ausführungen in Bezug auf die erfindungsgemäße Crossflow-Filtrationseinheit zur Diafiltration und insbesondere die Ausführungen in Bezug auf deren Retentatspalt und Permeatsammelspalt entsprechend. Das flächige Filtermaterial der CO-Einheit kann eine Mikrofiltrationsmembran und vorzugsweise eine Ultrafiltrationsmembran sein. Wie auch die erfindungsgemäße Crossflow-Filtrationseinheit, kann die CO-Einheit zu einer Filterkassette ausgebaut sein.

Das erfindungsgemäße Verfahren wird vorzugsweise unter den folgenden Bedingungen betrieben:

$$P_{DF} \geq P_{Retentat};$$

$x = V_{DF}/V_{Feed}$, vorzugsweise ist $x \geq 1$, besonders bevorzugt 3 bis 10; und
$k = V_{Feed}/V_{Retentat}$, vorzugsweise ist $k \geq 1$;

wobei

$P_{DF}$ der Druck ist, mit dem das Diafiltrationsmedium zugegeben wird,
$P_{Retentat}$ der Retentatausgangdruck ist, d. h. der Druck mit dem das Retentat die Filtrationsvorrichtung verlässt,
$V_{Retentat}$ der Volumenstrom des Retentats ist,
$V_{DF}$ der Volumenstrom des Diafiltrationsmediums ist,
$V_{Feed}$ der Volumenstrom der Speiseflüssigkeit ist,
$x$ das sogenannte DF-Verhältnis ist und
$k$ der sogenannte Konzentrationsfaktor ist.

Das erfindungsgemäße Verfahren eignet sich besonders für die Filtration, Diafiltration, Aufkonzentrierung und/oder Änderung der Ionenzusammensetzung einer Proteinlösung oder Kombinationen davon.

Das erfindungsgemäße Verfahren kann Bestandteil eines umfassenderen Verfahrensablaufes sein. Beispielsweise kann das Diafiltrationsverfahren einer Vorbehandlung nachgeschaltet und/oder einer Nachbehandlung vorgeschaltet sein. Beispielhaft für geeignete Vor- beziehungsweise Nachbehandlungen sind Umsetzen von Edukten zu Produkten auf biologischem oder chemischem Wege, thermische und mechanische Trennverfahren und chemische Analyseverfahren.

Die vorliegende Erfindung betrifft in einem weiteren Aspekt eine Vorrichtung zur Durchführung eines chemischen oder biologischen Verfahrens, welche die vorstehend beschriebene Crossflow-Diafiltrationseinheit umfasst. Eine derartige Vorrichtung kann beispielsweise einen Bioreaktor, eine Einheit zur Zellabtrennung, eine Einheit zur Diafiltration mit der erfindungsgemäßen Crossflow-Diafiltrationseinheit und eine Einheit zur Chromatographie umfassen. Geeignete chemische und biologische Verfahren sind beispielsweise die Herstellung von Impfstoffen oder Biopharmazeutika.

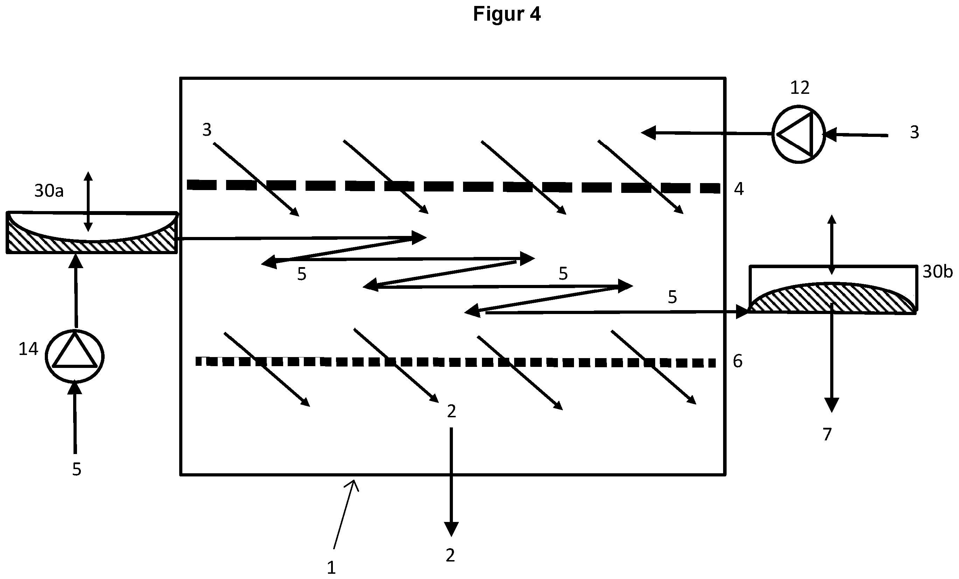

Darüber hinaus betrifft die vorliegende Erfindung die Verwendung der Crossflow-Filtrationseinheit für die kontinuierliche Diafiltration innerhalb eines chemischen oder biologischen Verfahrens, wobei dem Schritt der kontinuierlichen Diafiltration mindestens ein Aufbereitungsschritt für die Speiseflüssigkeit vorgelagert und/oder mindestens ein Nachbereitungsschritt für das Retentat nachgelagert ist. Geeignete Auf- bzw. Nachbereitungsschritte sind beispielsweise Umsetzen in einem Bioreaktor, gegebenenfalls gefolgt von Zellabtrennung, Chromatographie, Filtration, Aufkonzentrierung und Verdünnung (siehe Figur 4).

Die vorliegende Erfindung wird durch die folgenden Beispiele erläutert, ohne darauf beschränkt zu sein.

Beispiel 1

Eine Lösung von Albumin und NaCl (5,2 Gew.% Albumin in 0,9 Gew.% wässriger NaCl-Lösung) wurde einer Diafiltration unterzogen. Die Bestimmung der Albuminkonzentration erfolgte photometrisch bei einer Wellenlänge des Lichtes von 280 nm. Die Leitfähigkeit der Ausgangslösung betrug 14 mS. Als Diafiltrationsmedium wurde demineralisiertes Wasser verwendet.

Die Diafiltrationseinheit war als Filterkassette ausgebildet, wobei 13 Anordnungen von Diafiltrationspalt, flächigem ersten Filtermaterial, Retentatspalt, flächigem zweiten Filtermaterial, Permeatsammelspalt, flächigem zweiten Filtermaterial, Retentatspalt und flächigem ersten Filtermaterial übereinandergestapelt waren und von einem weiteren Diafiltrationsspalt ergänzt wurden. Als flächiges zweites Filtermaterial wurde eine Hydrosart® Membran des Typs 10 kDa der Sartorius Stedim Biotech GmbH verwendet. Als flächiges erstes Filtermaterial wurde eine Polyethersulfonmembran des Typs 30 kDa der Sartorius Stedim Biotech GmbH verwendet. Das DF-Verhältnis (Volumen Diafiltrationsmedium / Volumen Speiseflüssigkeit) betrug etwa 5,5 : 1.

Während der Versuchsdauer wurden die Einstellungen der Pumpen und Ventile nicht nachgeregelt. Die Diafiltrationseinheit arbeitete mit stabilen Leistungen. Die geringe Leitfähigkeit des Retentats von 2,2 mS zeigt an, dass eine hervorragende Diafiltrationsleistung erzielt wurde.

Das Ergebnis des vorstehenden Beispiels ist in der folgenden Tabelle 1 dargestellt.

Tabelle 1

| Zeit (min) | $P_{Feed}$ (bar) | $P_{Retentat}$ (bar) | $P_{DF}$ (bar) | $V_{Feed}$ (ml/min) | $V_{Retentat}$ (ml/min) | $V_{Permeat}$ ml/min | $C_{Protein}$ (Gew.%) | x (Vol/Vol) | $LF_{Permeat}$ (mS) | $LF_{Retentat}$ (mS) | T (°C) |
|---|---|---|---|---|---|---|---|---|---|---|---|
| 0 (Start) | 2,42 | 0,77 | 1,8 | 14,6 | 12,8 | 80,8 | 5,6 | 5,5 : 1 | 2,5 mS | 2,25 mS | ca. 20°C |
| 30 min | 2,40 | 0,77 | 1,8 | 14,6 | 14,3 | 82,0 | 4,96 | 5,5 : 1 | 2,4 mS | 2,18 mS | ca. 20°C |
| 60 min | 2,39 | 0,77 | 1,8 | 14,6 | 14,6 | 81 | 5,1 | 5,5 : 1 | 2,4 mS | 2,18 mS | ca. 20°C |

| $P_{Feed}$ | Druck, mit dem die Speiseflüssigkeit zugegeben wird |
| $P_{Retentat}$ | Retentatausgangsdruck |
| $P_{DF}$ | Druck, mit dem das Diafilrationsmedium zugegeben wird |
| $V_{Feed}$ | Volumenstrom der Speiseflüssigkeit |
| $V_{Retentat}$ | Volumenstrom des Retentats |
| $V_{Permeat}$ | Volumenstrom des Permeats |
| $C_{Protein}$ | Konzentration von Albumin (Gewichtsprozente) |
| x | DF-Verhältnis (Volumen Diafiltrationsmedium / Volumen Speiseflüssigkeit) |
| $LF_{Permeat}$ | Leitfähigkeit des Permeats |
| $LF_{Retentat}$ | Leitfähigkeit des Retentats |
| T | Temperatur |

Beispiel 2

Eine Lösung von ca. 22 g/l Albumin (1 g/l entspricht 0,1 Gew.-%) in 10 millimolarem KPI Puffer (10 mmol/l Kalimdihydrogenphosphat und 10 mmol/l Dikaliumhydrogenphosphat) und 0,9 Gew.-% NaCl wurde einer Diafiltration unterzogen. Die Bestimmung der Albuminkonzentration erfolgte photometrisch bei einer Wellenlänge des Lichtes von 280 nm. Die Leitfähigkeit der Ausgangslösung betrug 15,9 mS. Als Diafiltrationsmedium (DF) wurde 10 millimolare KPI Lösung mit einer Leitfähigkeit von 2,05 mS benutzt.

Die verwendeten Diafiltrationseinheiten waren als Filterkassette ausgebildet, wobei bei jeder der Diafiltrationseinheiten jeweils 8 Anordnungen von Diafiltrationspalt, flächigem ersten Filtermaterial, Retentatspalt, flächigem zweiten Filtermaterial, Permeatsammelspalt, flächigem zweiten Filtermaterial, Retentatspalt und flächigem ersten Filtermaterial übereinandergestapelt waren und von einem weiteren Diafiltrationsspalt ergänzt wurden. Als flächiges zweites Filtermaterial wurde eine Hydrosart® Membran des Typs 10 kDa der Sartorius Stedim Biotech GmbH verwendet. Als flächiges erstes Filtermaterial wurde eine Hydrosart® Membran des Typs 30 kDa der Sartorius Stedim Biotech GmbH verwendet.

In diesem Beispiel waren drei der vorstehend beschriebenen Diafiltrationseinheiten mit Hilfe von Umlenkplatten für die Speiseflüssigkeit so in Reihe geschaltet, dass die Speiseflüssigkeit seriell durch alle drei Kassetten geleitet wurde. Dagegen wurde die Diafiltrationsflüssigkeit parallel in alle drei Diafiltrationseinheiten geleitet.

Die Gesamt-Filterfläche aller drei flächigen zweiten Filtermaterialien betrug 0,2 m². Das DF-Verhältnis (Volumen Diafiltrationsmedium / Volumen Speiseflüssigkeit) betrug etwa 4,5:1

Das Ergebnis des vorstehenden Beispiels ist in der folgenden Tabelle 2 dargestellt.

Während der Versuchsdauer wurden die Einstellungen der Pumpen und Ventile nicht nachgeregelt. Die Diafiltrationseinheit arbeitete über einen Zeitraum von 2 Stunden mit konstanten und stabilen Leistungen.

Die Effektivität der Diafiltration wurde aus der Abnahme der Leitfähigkeit (LF) im Feedstrom berechnet und ist in der Tabelle als "Clearance" bezeichnet. Die Berechnung der Clearance in Prozent erfolgte nach der Formel

$$\text{Clearance} = 100 - ((LF_{Retentat} - LF_{DF}) / (LF_{Feed} - LF_{DF}) \ast 100),$$

wobei LF die Leitfähigkeit des jeweiligen Mediums ist.

Der so errechnete Clearance-Wert wurde mit dem theoretisch erreichbaren Wert verglichen.

Die Berechnung der theoretisch erreichbaren Clearance erfolgte nach der Formel:

$$\text{theoretisch erreichbare Clearance} = 100 - ((1/e)^n \cdot 100)$$

wobei e die Eulersche Zahl ist und n das DF-Verhältnis darstellt.

Beispiel 2 zeigt, dass mit der erfindungsgemäßen Vorrichtung eine äußerst effektive kontinuierliche Diafiltration möglich ist, die den theoretischen (maximal möglichen) Clearance-Wert von 99% erreichen kann.

Hierzu muss angemerkt werden, dass bei einer Verdünnung mit der im Beispiel genannten 4,5-fachen Menge an Diafiltrationslösung und einer anschließenden Aufkonzentrierung der Proteinlösung auf das ursprüngliche Volumen nach bekanntem Stand der Technik lediglich ein Clearance-Wert von 78% erreicht werden kann. Auch eine sukzessive Zugabe in 5 Einzelschritten mit jeweils anschließender Konzentrierung auf das Ausgangsvolumen ergäbe einen Clearance-Wert von lediglich 96%. Selbst dieser liegt noch deutlich unterhalb der erfindungsgemäß erzielbaren Clearance von 99%.

Tabelle 2

| Zeit (min) | $P_{Feed}$ (bar) | $P_{Retentat}$ (bar) | $P_{DF}$ (bar) | $V_{Feed}$ (ml/min) | $V_{Retentat}$ (ml/min) | $V_{Permeat}$ ml/min | $c_{Protein}$ (g/l) | x (Vol/Vol) | $LF_{Retentat}$ (mS) | Clearance % | Clearance theoretisch % | T (°C) |
|---|---|---|---|---|---|---|---|---|---|---|---|---|
| 5 min | 1,07 | 1,08 | 1,2 | 20 | 20 | 90 | 21,3 | 4,5 | 2,28 | 99 | 99 | ca. 19°C |
| 15 min | 1,09 | 1,02 | 1,2 | 22 | 20 | 90 | 22,4 | 4,5 | 2,18 | 99 | 99 | ca. 19°C |
| 30 min | 1,07 | 1,01 | 1,19 | 22 | 20 | 88 | 21,5 | 4,5 | 2,17 | 99 | 99 | ca. 19°C |
| 60 min | 1,03 | 0,97 | 1,14 | 21,5 | 20 | 88 | 23,6 | 4,5 | 2,14 | 99 | 99 | ca. 19°C |
| 90min | 1,01 | 0,95 | 1,12 | 21 | 20 | 88 | 25 | 4,5 | 2,16 | 99 | 99 | ca. 19°C |
| 120 min | 1,00 | 0,95 | 1,12 | 22 | 20 | 88 | 22,5 | 4,5 | 2,16 | 99 | 99 | ca. 19°C |

| $P_{Feed}$ | Druck, mit dem die Speiseflüssigkeit (Feed) zugegeben wird |
|---|---|
| $P_{Retentat}$ | Retentatausgangsdruck |
| $P_{DF}$ | Druck, mit dem das Diafilrationsmedium zugegeben wird |
| $V_{Feed}$ | Volumenstrom der Speiseflüssigkeit |
| $V_{Retentat}$ | Volumenstrom des Retentats |
| $V_{Permeat}$ | Volumenstrom des Permeats |
| $c_{Protein}$ | Konzentration von Albumin (g/l) |
| x | DF-Verhältnis (Volumen Diafiltrationsmedium / Volumen Speiseflüssigkeit) |
| $LF_{Retentat}$ | Leitfähigkeit des Retentats |
| Clearance | Leitfähigkeitsabnahme in Prozent (100% = maximal mögliche Leitfähigkeitsabnahme) |
| Clearance theoretisch | maximal mögliche Leitfähigkeitsabnahme in Prozent |
| T | Temperatur |

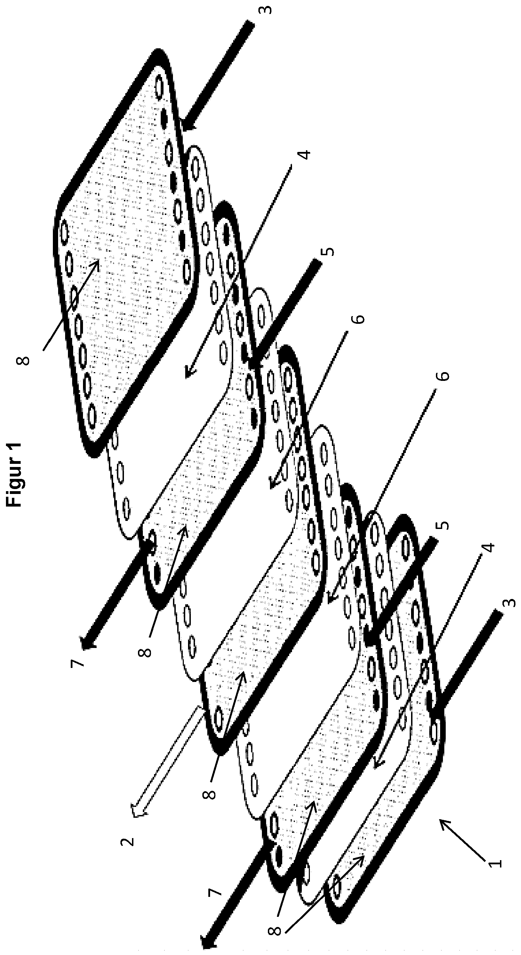

Figur 1 zeigt einen möglichen Aufbau einer erfindungsgemäßen Crossflow-Diafiltrationseinheit (1) mit einem flächigen zweiten Filtermaterial in Form einer Ultrafiltrationsmebran (6) und einem flächigen ersten Filtermaterial in Form einer Mikrofiltrationsmembran (4), wobei die Ströme von Diafiltrationsmedium (3), Speiseflüssigkeit (5), Retentat (7) und Permeat (2) durch Pfeile veranschaulicht sind. Der Diafiltrationsspalt, der Retentatspalt und der Permeatsammelspalt werden durch Abstandshalter (8) für die entsprechenden Medien offengehalten.

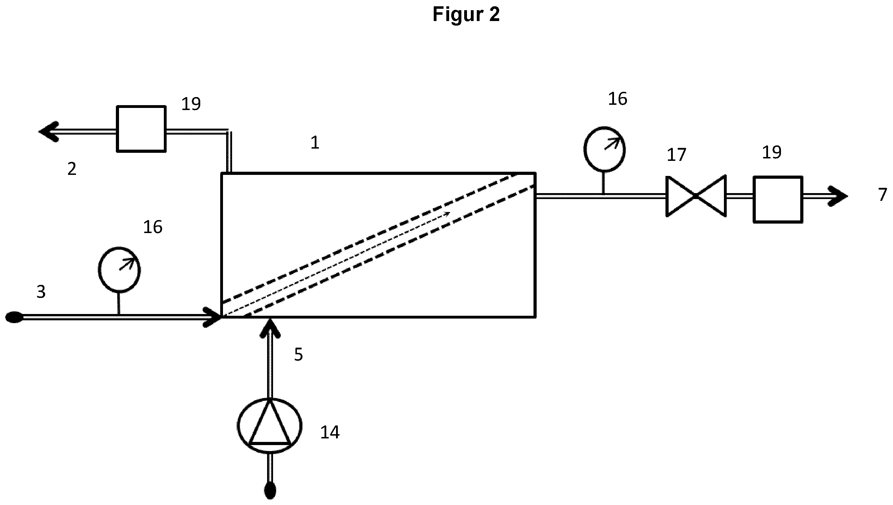

Figur 2 zeigt beispielhaft schematisch die Durchführung des erfindungsgemäßen Diafiltrationsverfahrens mit einer Crossflow-Diafiltrationseinheit, welche als Diafiltrationskassette (1) ausgebildet ist. Dabei wird am Einlass für die Speiseflüssigkeit die Speiseflüssigkeit (5) über eine Pumpe (14) zugeführt. Vor dem Einlass für das Diafiltrationsmedium und nach dem Auslass für das Retentat wird der Druck des Diafiltrationsmediums (3) beziehungsweise des Retentats (7) durch Manometer (16) gemessen. Die jeweilige Zusammensetzung des Retentats (7) sowie des Permeats (2) wird durch eine Messeinrichtung, beispielsweise ein Konduktometer (19), überwacht. Der Volumenstrom des Retentats (7) wird durch ein Drosselventil (17) gesteuert.

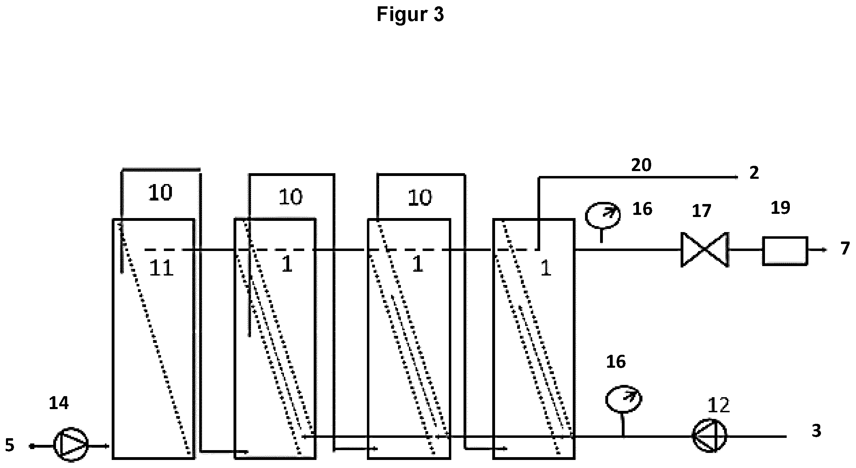

Figur 3 zeigt beispielhaft schematisch die Durchführung des erfindungsgemäßen Diafiltrationsverfahrens, wobei drei Diafiltrationskassetten (1) in Reihe geschaltet sind. Vorgeschaltet ist ein Schritt der Auftrennung einer Flüssigkeit in ein Vorretentat und ein Vorpermeat mittels einer gewöhnlichen Crossflow-Filtrationskassette (11). Die Speiseflüssigkeit (5) wird über eine Pumpe (14) zugeführt. Das Diafiltrationsmedium (3) wird den drei Diafiltrationskassetten (1) über eine Pumpe (12) zugeführt. Das Vorretentat aus der jeweiligen vorgeschalteten Filtrationseinheit wird über Kanal (10) der jeweiligen nachgeschalteten Filtrationseinheit als Speiseflüssigkeit zugeführt. Vor dem Einlass für das Diafiltrationsmedium und nach dem Auslass für das Retentat wird der Druck des Diafiltrationsmediums (3) beziehungsweise des Retentats (7) durch Manometer (16) gemessen. Die Zusammensetzung des Retentats (7) wird durch eine Messeinrichtung, beispielsweise ein Konduktometer (19), überwacht. Der Volumenstrom des Retentats (7) wird durch ein Drosselventil (17) gesteuert. Das Permeat (2) aus allen Modulen (11, 1) wird über die Sammelleitung (20) abgeführt.

Figur 4 zeigt beispielhaft schematisch die Durchführung des erfindungsgemäßen Diafiltrationsverfahrens sowie eine Crossflow-Diafiltrationseinheit gemäß der vorliegenden Erfindung, wobei die Speiseflüssigkeit/das Retentat (5) im Retentatspalt oszilliert wird/werden. Die Speiseflüssigkeit (5) wird über eine Pumpe (14) in ein Reservoir (30a) und nachfolgend in den Retentatspalt eingebracht. Danach durchläuft die Speiseflüssigkeit/das Retentat (5) eine zweite Vorrichtung zur Erzeugung von Oszillation bzw. ein zweites Reservoir (30b). Das Diafiltrationsmedium (3) wird über eine Pumpe (12) in den Diafiltrationsspalt eingebracht. Die dargestellte Crossflow-Diafiltrationseinheit weist zwei Vorrichtungen zur Erzeugung von Oszillation auf, wobei die erste Vorrichtung ein erstes Reservoir (30a) umfasst und die zweite Vorrichtung ein davon verschiedenes zweites Reservoir (30b) umfasst. Reservoir (30a) ist fluidleitend mit dem Einlass für die Speiseflüssigkeit (5) verbunden. Reservoir (30b) ist fluidleitend mit dem Auslass für das Retentat (7) verbunden. Jedes Reservoir ist durch eine elastische und fluidundurchlässige Membran in zwei Hälften getrennt. Die erste Hälfte des ersten Reservoirs (30a) (in Figur 4 nicht schraffiert dargestellt) ist über eine Ventilsteuerung mit einer Druckquelle verbunden. Die zweite Hälfte des ersten Reservoirs (30a) (in Figur 4 schraffiert dargestellt) ist fluidleitend mit dem Einlass für die Speiseflüssigkeit verbunden. Das zweite Reservoir (30b) ist durch eine elastische und fluidundurchlässige Membran in zwei Hälften getrennt. Die erste Hälfte des zweiten Reservoirs (30b) (in Figur 4 nicht schraffiert dargestellt) ist über eine Ventilsteuerung mit einer Druckquelle verbunden. Die zweite Hälfte des zweiten Reservoirs (30b) (in Figur 4 schraffiert dargestellt) ist fluidleitend mit dem Auslass für das Retentat verbunden. Durch Steuerung beziehungsweise Regelung des Druckniveaus der Druckluft (gegenläufiges Beaufschlagen der jeweils ersten Hälfte von 30a und 30b mit Druckluft) wird eine oszillierende Bewegung der Speiseflüssigkeit/des Retentats im Rententatspalt erzeugt. Ein Teil des Retentats wird kontinuierlich abgeführt, um eine kontinuierliche Verfahrensführung zu gewährleisten.

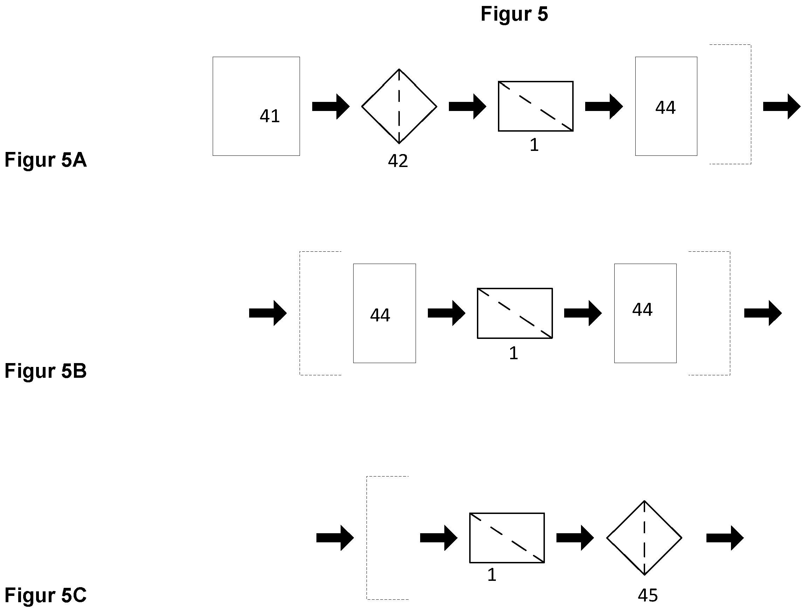

Figur 5 zeigt beispielhaft verschiedene Ausschnitte A bis C von Prozessschemata zur Durchführung eines Verfahrens zur Herstellung von Biopharmazeutika, wobei die Prozesse jeweils die Bereitstellung mindestens einer erfindungsgemäßen Crossflow-Diafiltrationseinheit (1) umfassen. Durch die gestrichelten eckigen Klammern und die vor- bzw. nachstehenden Pfeile wird angezeigt, dass weitere Verfahrensschritte vor- bzw. nachgeschaltet sein können.

Die in Figur 5A dargestellte Ausführungsform umfasst eine erfindungsgemäße Crossflow-Diafiltrationseinheit (1), wobei ein Bioreaktor (41) sowie eine Zellabtrennungseinheit (42) vorgeschaltet sind. Der Crossflow-Diafiltrationseinheit (1) nachgeschaltet ist eine Chromatographieeinheit / ein Chromatographieschritt (44). Der Strom der Produktlösung aus Bioreaktor (1) ist durch Pfeile veranschaulicht.

Die in Figur 5B dargestellte Ausführungsform umfasst eine erfindungsgemäße Crossflow-Diafiltrationseinheit (1) zwischen zwei Chromatographieschritten (44), d. h. der Diafiltration ist ein Chromatographieschritt vorgeschaltet und ein Chromatographieschritt nachgeschaltet.

Die in Figur 5C dargestellte Ausführungsform umfasst eine erfindungsgemäße Crossflow-Diafiltrationseinheit (1) vor einem finalen Filtrationsschritt (45).

Die in Figuren 5A bis 5C dargestellten Prozessschritte können beliebig um weitere Prozessschritte erweitert oder beliebig kombiniert werden. Die erfindungsgemäße Crossflow-Filtration wird dabei vorzugsweise zur Filtration, Diafiltration, Aufkonzentrierung und/oder Änderung der Zusammensetzung einer Lösung eingesetzt.

Bezugszeichenliste

| 1 | Crossflow-Diafiltrationseinheit/Diafiltrationskassette |
|---|---|
| 2 | Permeat |
| 3 | Diafiltrationsmedium |
| 4 | Erstes Filtermaterial |
| 5 | Speiseflüssigkeit |
| 6 | Zweites Filtermaterial |
| 7 | Retentat |
| 8 | Abstandshalter |
| 10 | Kanal zur Umlenkung der Speiseflüssigkeit |
| 11 | Standard Crossflow Cassette |
| 12 | Diafiltrations-Pumpe |
| 14 | Speiseflüssigkeits-Pumpe |
| 16 | Manometer |
| 17 | Drosselventil |
| 19 | Konduktometer |
| 20 | Sammelleitung |
| 30a, 30b | Reservoir |
| 41 | Bioreaktor |
| 42 | Zellabtrennung |
| 44 | Chromatographie |
| 45 | Filtrationsschritt/Filter |

Weiterhin betrifft die vorliegende Erfindung die folgenden Punkte 1 bis 13:

1. Crossflow-Filtrationseinheit (1) zur kontinuierlichen Diafiltration einer Speiseflüssigkeit (5) zum Erhalten eines Retentats (7) und eines Permeats (2), mindestens umfassend

einen Diafiltrationspalt,
ein flächiges erstes Filtermaterial (4),
einen Retentatspalt,

ein flächiges zweites Filtermaterial (6) und
einen Permeatsammelspalt,

derart angeordnet, dass das flächige erste Filtermaterial (4) den Diafiltrationspalt und den Retentatspalt voneinander abgrenzt und das flächige zweite Filtermaterial (6) den Retentatspalt und den Permeatsammelspalt voneinander abgrenzt,
wobei
der Diafiltrationspalt fluidleitend mit mindestens einem Einlass für das Diafiltrationsmedium (3), der Retentatspalt fluidleitend mit mindestens einem Einlass für die Speiseflüssigkeit (5) und mit mindestens einem Auslass für das Retentat (7) verbunden ist und der Permeatsammelspalt fluidleitend mit mindestens einem Auslass für das Permeat (2) verbunden ist und wobei die Porengröße beziehungsweise die Molekulargewichtsausschlussgrenze des flächigen ersten Filtermaterials (4) mindestens so groß wie die Porengröße beziehungsweise die Molekulargewichtsausschlussgrenze des flächigen zweiten Filtermaterials (6) ist.

2. Crossflow-Filtrationseinheit (1) nach Punkt 1,
wobei das flächige erste Filtermaterial (4) eine erste Filtrationsmembran ist und/oder das flächige zweite Filtermaterial (6) eine zweite Filtrationsmembran ist.

3. Crossflow-Filtrationseinheit (1) nach Punkt 2,
wobei die erste Filtrationsmembran eine Mikro- oder Ultrafiltrationsmembran ist und/oder die zweite Filtrationsmembran eine Ultrafiltrationsmembran ist.

4. Crossflow-Filtrationseinheit (1) nach einem der Punkte 1 bis 3,
wobei die Crossflow-Filtrationseinheit (1) eine Mehrzahl übereinandergestapelter Anordnungen von Diafiltrationspalt, flächigem ersten Filtermaterial (4), Retentatspalt, flächigem zweiten Filtermaterial (6), Permeatsammelspalt, flächigem zweiten Filtermaterial (6), Retentatspalt und flächigem ersten Filtermaterial (4) aufweist, sodass die gestapelten Anordnungen zu einer Filterkassette zusammengefasst sind.

5. Crossflow-Filtrationseinheit (1) nach einem der Punkte 1 bis 4, wobei
das freie Volumen des Diafiltrationspaltes und/oder des Retentatspaltes in der Flussrichtung vom Einlass für die Speiseflüssigkeit (5) zum Auslass für das Retentat (7) abnimmt.

6. Crossflow-Filtrationseinheit (1) nach Punkt 5,
wobei im Retentatspalt mehrere Lagen textiler Materialien übereinander angeordnet sind, derart, dass das freie Volumen des Retentatspaltes in Flussrichtung abnimmt.

7. Verfahren zur Diafiltration einer Speiseflüssigkeit (5) zum Erhalten eines Retentats (7) und eines Permeats (2), umfassend die Schritte

(A) Bereitstellen einer Crossflow-Filtrationseinheit (1) nach einem der Punkte 1 bis 6;
(B) Zuführen eines Diafiltrationsmediums (3) in den Einlass für das Diafiltrationsmedium (3);
(C) Zuführen der Speiseflüssigkeit (5) in den Einlass für die Speiseflüssigkeit (5);
(D) Abführen des Retentats (7) aus dem Auslass für das Retentat (7); und
(E) Abführen des Permeats (2) aus dem Auslass für das Permeat (2).

8. Verfahren zur Diafiltration nach Punkt 7, wobei
das Diafiltrationsmedium (3) und die Speiseflüssigkeit (5) kontinuierlich zugeführt werden.

9. Verfahren zur Diafiltration nach Punkt 7 oder 8, wobei

in Schritt (A) mehrere Crossflow-Filtrationseinheiten (1), jeweils nach einem der Punkte 1 bis 6, bereitgestellt werden und so in Reihe geschaltet werden, dass der Auslass für das Retentat (7) der jeweiligen vorgeschalteten Crossflow-Filtrationseinheit (1) fluidleitend mit dem Einlass für die Speiseflüssigkeit (5) einer nachgeschalteten Crossflow-Filtrationseinheit (1) verbunden ist, und
in Schritt (C) die Speiseflüssigkeit (5) in den Einlass für die Speiseflüssigkeit (5) derjenigen Crossflow-Filtrationseinheit (1), welcher keine andere Crossflow-Filtrationseinheit (1) vorgeschaltet ist, zugeführt wird und
in Schritt (D) das Retentat (7) aus dem Auslass für das Retentat (7) derjenigen Crossflow-Filtrationseinheit (1), welcher keine andere Crossflow-Filtrationseinheit (1) nachgeschaltet ist, abgeführt wird.

10. Verfahren zur Diafiltration nach einem der Punkte 7 bis 9, wobei
das in Schritt (D) abgeführte Retentat (7) zumindest teilweise in den Einlass für die Speiseflüssigkeit (5) zurückgeführt wird.

11. Verfahren zur Diafiltration nach einem der Punkte 7 bis 10, weiter umfassend den Schritt:
(C0) Auftrennung einer Flüssigkeit in ein Vorretentat und ein Vorpermeat, wobei das Vorretentat oder das Vorpermeat als Speiseflüssigkeit (5) verwendet wird.

12. Verwendung der Crossflow-Filtrationseinheit (1) nach einem der Punkte 1 bis 6 für die Filtration, Diafiltration, Aufkonzentrierung und/oder Änderung der Ionenzusammensetzung einer Lösung.

13. Verwendung der Crossflow-Filtrationseinheit (1) nach einem der Punkte 1 bis 6 für die kontinuierliche Diafiltration innerhalb eines chemischen oder biologischen Verfahrens, wobei dem Schritt der kontinuierlichen Diafiltration mindestens ein Aufbereitungsschritt für die Speiseflüssigkeit (5) vorgelagert und/oder mindestens ein Nachbereitungsschritt für das Retentat (7) nachgelagert ist.

**Patentansprüche**

1. Verfahren zur Diafiltration einer Speiseflüssigkeit (5) zum Erhalten eines Retentats (7) und eines Permeats (2), umfassend die Schritte

(A) Bereitstellen einer Crossflow-Filtrationseinheit (1) zur kontinuierlichen Diafiltration, mindestens umfassend

einen Diafiltrationspalt,
ein flächiges erstes Filtermaterial (4),
einen Retentatspalt,
ein flächiges zweites Filtermaterial (6) und
einen Permeatsammelspalt,
derart angeordnet, dass das flächige erste Filtermaterial (4) den Diafiltrationspalt und den Retentatspalt voneinander abgrenzt und das flächige zweite Filtermaterial (6) den Retentatspalt und den Permeatsammelspalt voneinander abgrenzt,
wobei
der Diafiltrationspalt fluidleitend mit mindestens einem Einlass für das Diafiltrationsmedium (3), der Retentatspalt fluidleitend mit mindestens einem Einlass für die Speiseflüssigkeit (5) und mit mindestens einem Auslass für das Retentat (7) verbunden ist und der Permeatsammelspalt fluidleitend mit mindestens einem Auslass für das Permeat (2) verbunden ist, und wobei die Porengröße beziehungsweise die Molekulargewichtsausschlussgrenze des flächigen ersten Filtermaterials (4) mindestens so groß wie die Porengröße beziehungsweise die Molekulargewichtsausschlussgrenze des flächigen zweiten Filtermaterials (6) ist;

(B) Zuführen eines Diafiltrationsmediums (3) in den Einlass für das Diafiltrationsmedium (3);
(C) Zuführen der Speiseflüssigkeit (5) in den Einlass für die Speiseflüssigkeit (5);
(D) Abführen des Retentats (7) aus dem Auslass für das Retentat (7); und
(E) Abführen des Permeats (2) aus dem Auslass für das Permeat (2),
wobei das Verfahren unter den folgenden Bedingungen betrieben wird:

$$P_{DF} \geq P_{Retentat};$$

$x \geq 1$, wobei $x = V_{DF}/V_{Feed}$; und
$k \geq 1$, wobei $k = V_{Feed}/V_{Retentat}$;
wobei
$P_{DF}$ der Druck ist, mit dem das Diafiltrationsmedium in Schritt (B) zugeführt wird,
$P_{Retentat}$ der Retentatausgangdruck ist, d. h. der Druck mit dem das Retentat die Filtrationseinheit (1) in Schritt (D) verlässt,
$V_{Retentat}$ der Volumenstrom des Retentats ist,
$V_{DF}$ der Volumenstrom des Diafiltrationsmediums ist, und
$V_{Feed}$ der Volumenstrom der Speiseflüssigkeit ist.

**2.** Verfahren zur Diafiltration nach Anspruch 1, wobei das flächige erste Filtermaterial (4) der Crossflow-Filtrationseinheit (1) eine erste Filtrationsmembran ist und/oder das flächige zweite Filtermaterial (6) der Crossflow-Filtrationseinheit (1) eine zweite Filtrationsmembran ist.

**3.** Verfahren zur Diafiltration nach Anspruch 2, wobei die erste Filtrationsmembran der Crossflow-Filtrationseinheit (1) eine Mikro- oder Ultrafiltrationsmembran ist und/oder die zweite Filtrationsmembran der Crossflow-Filtrationseinheit (1) eine Ultrafiltrationsmembran ist.

**4.** Verfahren zur Diafiltration nach einem der Ansprüche 1 bis 3, wobei die Crossflow-Filtrationseinheit (1) eine Mehrzahl übereinandergestapelter Anordnungen von Diafiltrationspalt, flächigem ersten Filtermaterial (4), Retentatspalt, flächigem zweiten Filtermaterial (6), Permeatsammelspalt, flächigem zweiten Filtermaterial (6), Retentatspalt und flächigem ersten Filtermaterial (4) aufweist, sodass die gestapelten Anordnungen zu einer Filterkassette zusammengefasst sind.

**5.** Verfahren zur Diafiltration nach einem der Ansprüche 1 bis 4, wobei das freie Volumen des Diafiltrationspaltes und/oder des Retentatspaltes der Crossflow-Filtrationseinheit (1) in der Flussrichtung vom Einlass für die Speiseflüssigkeit (5) zum Auslass für das Retentat (7) abnimmt.

**6.** Verfahren zur Diafiltration nach Anspruch 5, wobei im Retentatspalt der Crossflow-Filtrationseinheit (1) mehrere Lagen textiler Materialien übereinander angeordnet sind, derart, dass das freie Volumen des Retentatspaltes in Flussrichtung abnimmt.

**7.** Verfahren zur Diafiltration nach einem der Ansprüche 1 bis 6, wobei das Diafiltrationsmedium (3) und die Speiseflüssigkeit (5) kontinuierlich zugeführt werden.

**8.** Verfahren zur Diafiltration nach einem der Ansprüche 1 bis 7, wobei

in Schritt (A) mehrere der Crossflow-Filtrationseinheiten (1) bereitgestellt werden und so in Reihe geschaltet werden, dass der Auslass für das Retentat (7) der jeweiligen vorgeschalteten Crossflow-Filtrationseinheit (1) fluidleitend mit dem Einlass für die Speiseflüssigkeit (5) einer nachgeschalteten Crossflow-Filtrationseinheit (1) verbunden ist, und
in Schritt (C) die Speiseflüssigkeit (5) in den Einlass für die Speiseflüssigkeit (5) derjenigen Crossflow-Filtrationseinheit (1), welcher keine andere Crossflow-Filtrationseinheit (1) vorgeschaltet ist, zugeführt wird und
in Schritt (D) das Retentat (7) aus dem Auslass für das Retentat (7) derjenigen Crossflow-Filtrationseinheit (1), welcher keine andere Crossflow-Filtrationseinheit (1) nachgeschaltet ist, abgeführt wird.

**9.** Verfahren zur Diafiltration nach einem der Ansprüche 1 bis 8, wobei das in Schritt (D) abgeführte Retentat (7) zumindest teilweise in den Einlass für die Speiseflüssigkeit (5) zurückgeführt wird.

**10.** Verfahren zur Diafiltration nach einem der Ansprüche 1 bis 9, weiter umfassend den Schritt:
(C0) Auftrennung einer Flüssigkeit in ein Vorretentat und ein Vorpermeat, wobei das Vorretentat oder das Vorpermeat als Speiseflüssigkeit (5) verwendet wird.

**11.** Verfahren zur Diafiltration nach einem der Ansprüche 1 bis 10, wobei durch das Verfahren eine Lösung filtriert, diafiltriert, aufkonzentriert und/oder die Ionenzusammensetzung der Lösung verändert wird.

**12.** Verfahren zur Diafiltration nach einem der Ansprüche 1 bis 11, wobei das Verfahren einer Vorbehandlung nachgeschaltet und/oder einer Nachbehandlung vorgeschaltet ist.

**13.** Verfahren zur Diafiltration nach einem der Ansprüche 1 bis 12, wobei x 3 bis 10 beträgt.


**Claims**

**1.** Method for diafiltration of a feed fluid (5) to obtain a retentate (7) and a permeate (2), the method comprising the steps of:

(A) providing a crossflow filtration unit (1) for continuous diafiltration, at least comprising:

a diafiltration channel,
a flat first filter material (4),
a retentate channel,
a flat second filter material (6) and
a permeate collection channel,
arranged such that the flat first filter material (4) delimits the diafiltration channel and the retentate channel from one another, and the flat second filter material (6) delimits the retentate channel and the permeate collection channel from one another,
wherein
the diafiltration channel is connected in a fluid-conducting manner to at least one inlet for the diafiltration medium (3), the retentate channel is connected in a fluid-conducting manner to at least one inlet for the feed fluid (5) and to at least one outlet for the retentate (7), and the permeate collection channel is connected in a fluid-conducting manner to at least one outlet for the permeate (2), and wherein the pore size or the molecular weight cut-off of the flat first filter material (4) is at least as large as the pore size or the molecular weight cut-off of the flat second filter material (6);

(B) feeding a diafiltration medium (3) into the inlet for the diafiltration medium (3);
(C) feeding the feed fluid (5) into the inlet for the feed fluid (5);
(D) discharging the retentate (7) from the outlet for the retentate (7); and
(E) discharging the permeate (2) from the outlet for the permeate (2),
wherein the method is operated under the following conditions:

$$P_{DF} \geq P_{Retentate};$$

$x \geq 1$, where $x = V_{DF}/V_{Feed}$; and
$k \geq 1$, where $k = V_{Feed}/V_{Retentate}$;
where
$P_{DF}$ is the pressure at which the diafiltration medium is fed in step (B),
$P_{Retentate}$ is the retentate output pressure, i.e. the pressure at which the retentate leaves the filtration unit (1) in step (D),
$V_{Retentate}$ is the volume flow of the retentate,
$V_{DF}$ is the volume flow of the diafiltration medium, and
$V_{Feed}$ is the volume flow of the feed fluid.

2. Method for diafiltration according to claim 1, wherein the flat first filter material (4) of the crossflow filtration unit (1) is a first filtration membrane, and/or the flat second filter material (6) of the crossflow filtration unit (1) is a second filtration membrane.

3. Method for diafiltration according to claim 2, wherein the first filtration membrane of the crossflow filtration unit (1) is a microfiltration membrane or an ultrafiltration membrane, and/or the second filtration membrane of the crossflow filtration unit (1) is an ultrafiltration membrane.

4. Method for diafiltration according to any one of claims 1 to 3, wherein the crossflow filtration unit (1) comprises a plurality of stacked arrays of diafiltration channel, flat first filter material (4), retentate channel, flat second filter material (6), permeate collection channel, flat second filter material (6), retentate channel, and flat first filter material (4), such that the stacked arrays are combined to form a filter cassette.

5. Method for diafiltration according to any one of claims 1 to 4, wherein the free volume of the diafiltration channel and/or the retentate channel of the crossflow filtration unit (1) decreases in the flow direction from the inlet for the feed fluid (5) to the outlet for the retentate (7).

6. Method for diafiltration according to claim 5, wherein a plurality of layers of textile materials are arranged one above another in the retentate channel of the crossflow filtration unit (1) such that the free volume of the retentate channel decreases in the flow direction.

7. Method for diafiltration according to any one of claims 1 to 6, wherein the diafiltration medium (3) and the feed fluid (5) are fed in continuously.

8. Method for diafiltration according to any one of claims 1 to 7, wherein

    in step (A) a plurality of crossflow filtration units (1) are provided and are connected in series such that the outlet for the retentate (7) of the respective upstream crossflow filtration unit (1) is connected in a fluid-conducting manner to the inlet for the feed fluid (5) of a downstream crossflow filtration unit (1), and
    in step (C) the feed fluid (5) is fed into the inlet for the feed fluid (5) of that crossflow filtration unit (1) which is not preceded by any other crossflow filtration unit (1) and
    in step (D) the retentate (7) is discharged from the outlet for the retentate (7) of that crossflow filtration unit (1) which is not followed by any other crossflow filtration unit (1) .

9. Method for diafiltration according to any one of claims 1 to 8, wherein the retentate (7) discharged in step (D) is recycled at least partially into the inlet for the feed fluid (5).

10. Method for diafiltration according to any one of claims 1 to 9, further comprising the step of:
    (C0) separating a fluid into a pre-retentate and a pre-permeate, the pre-retentate or the pre-permeate being used as the feed fluid (5).

11. Method for diafiltration according to any one of claims 1 to 10, wherein the method filters, diafilters, concentrates a solution and/or changes the ion composition of the solution.

12. Method for diafiltration according to any one of claims 1 to 11, wherein the method follows a pre-treatment and/or precedes an aftertreatment.

13. Method for diafiltration according to any one of claims 1 to 12, wherein x is 3 to 10.


**Revendications**

1. Procédé de diafiltration d'un liquide d'alimentation (5) pour obtenir un rétentat (7) et un perméat (2), comprenant les étapes de :

    (A) fourniture d'une unité de filtration à flux tangentiel (1) pour une diafiltration en continu, comprenant au moins

        une fente de diafiltration,
        un premier matériau filtrant (4) plat,
        une fente de rétentat,
        un second matériau filtrant (6) plat et
        une fente collectrice de perméat,
        agencée de sorte que le premier matériau filtrant (4) plat délimite la fente de diafiltration et la fente de rétentat l'une par rapport à l'autre et le second matériau filtrant (6) plat délimite la fente de rétentat et la fente collectrice de perméat l'une par rapport à l'autre,
        dans lequel
        la fente de diafiltration est reliée de façon à conduire du liquide avec au moins une admission pour le milieu de diafiltration (3), la fente de rétentat est reliée de façon à conduire du liquide avec au moins une admission pour le liquide d'alimentation (5) et avec au moins une évacuation pour le rétentat (7), et la fente collectrice de perméat est reliée de façon à conduire du liquide avec au moins une admission pour le perméat (2), et dans lequel la taille de pore, respectivement la limite d'exclusion de poids moléculaire, du premier matériau filtrant (4) plat est au moins aussi grande que la taille de pore, respectivement la limite d'exclusion de poids moléculaire, du second matériau filtrant (6) plat;

    (B) amenée d'un milieu de diafiltration (3) jusque dans l'admission pour le milieu de diafiltration (3);
    (C) amenée du liquide d'alimentation (5) jusque dans l'admission pour le liquide d'alimentation (5);
    (D) évacuation du rétentat (7) hors de l'admission pour le rétentat (7); et
    (E) évacuation du perméat (2) hors de l'admission pour le perméat (2),
    dans lequel le procédé est exploité dans les conditions suivantes :

$$P_{DF} \geq P_{Rétentat};$$

$x \geq 1$, dans lequel $x = V_{DF}/V_{Feed}$; et

$k \geq 1$, dans lequel $k = V_{Feed}/V_{Rétentat}$;

dans lequel

$P_{DF}$ est la pression sous laquelle le milieu de diafiltration est amené dans l'étape B),

$P_{Rétentat}$ est la pression de sortie de rétentat, c'est-à-dire la pression sous laquelle le rétentat quitte l'unité de filtration à flux tangentiel (1) dans l'étape D),

$V_{Rétentat}$ est le débit volumétrique du rétentat,

$V_{DF}$ est le débit volumétrique du milieu de diafiltration, et

$V_{Feed}$ est le débit volumétrique du liquide d'alimentation.

2. Procédé de diafiltration selon la revendication 1, dans lequel le premier matériau filtrant (4) plat de l'unité de filtration à flux tangentiel (1) est une première membrane de filtration et/ou le second matériau filtrant (6) plat de l'unité de filtration à flux tangentiel (1) est une seconde membrane de filtration.

3. Procédé de diafiltration selon la revendication 2, dans lequel la première membrane de filtration de l'unité de filtration à flux tangentiel (1) est une membrane de microfiltration ou d'ultrafiltration et/ou la seconde membrane de filtration de l'unité de filtration à flux tangentiel (1) est une membrane d'ultrafiltration.

4. Procédé de diafiltration selon l'une des revendications 1 à 3, dans lequel l'unité de filtration à flux tangentiel (1) présente une pluralité d'agencements, empilés les uns sur les autres, de fente de diafiltration, de premier matériau filtrant (4) plat, de fente de rétentat, de second matériau filtrant (6) plat, de fente collectrice de perméat, de second matériau filtrant (6) plat, de fente de rétentat et de premier matériau filtrant (4) plat, de sorte que les agencements empilés soient rassemblés pour former une cartouche filtrante.

5. Procédé de diafiltration selon l'une des revendications 1 à 4, dans lequel le volume libre de la fente de diafiltration et/ou de la fente de rétentat de l'unité de filtration à flux tangentiel (1) diminue dans la direction de flux depuis l'admission pour le liquide d'alimentation (5) jusqu'à l'évacuation pour le rétentat (7).

6. Procédé de diafiltration selon la revendication 5, dans lequel plusieurs couches de matériaux textiles sont agencées les unes sur les autres dans la fente de rétentat de l'unité de filtration à flux tangentiel (1), de sorte que le volume libre de la fente de rétentat diminue dans la direction de flux.

7. Procédé de diafiltration selon l'une des revendications 1 à 6, dans lequel le milieu de diafiltration (3) et le liquide d'alimentation (5) sont amenés en continu.

8. Procédé de diafiltration selon l'une des revendications 1 à 7, dans lequel dans l'étape (A) plusieurs parmi les unités de filtration à flux tangentiel (1) sont fournies et montées en série de sorte que l'évacuation pour le rétentat (7) de l'unité de filtration à flux tangentiel (1) respective montée en amont soit reliée de façon à conduire du liquide avec l'admission pour le liquide d'alimentation (5) d'une unité de filtration à flux tangentiel (1) montée en aval, et

dans l'étape (C) le liquide d'alimentation (5) est amené jusque dans l'admission pour le liquide d'alimentation (5) de l'unité de filtration à flux tangentiel (1) pour laquelle aucune autre unité de filtration à flux tangentiel (1) n'est montée en amont, et

dans l'étape (D) le rétentat (7) est évacué hors de l'évacuation pour le rétentat (7) de l'unité de filtration à flux tangentiel (1) pour laquelle aucune autre unité de filtration à flux tangentiel (1) n'est montée en aval.

9. Procédé de diafiltration selon l'une des revendications 1 à 8, dans lequel le rétentat (7) évacué dans l'étape (D) est renvoyé au moins partiellement jusque dans l'admission pour le liquide d'alimentation (5).

10. Procédé de diafiltration selon l'une des revendications 1 à 9, comprenant en outre l'étape de :

(C0) séparation d'un liquide en un rétentat amont et un perméat amont, dans lequel le rétentat amont ou le perméat amont est utilisé en tant que liquide d'alimentation (5).

11. Procédé de diafiltration selon l'une des revendications 1 à 10, dans lequel, via le procédé, une solution est filtrée, diafiltrée, augmentée en concentration et/ou la composition ionique de la solution est modifiée.

12. Procédé de diafiltration selon l'une des revendications 1 à 11, dans lequel le procédé est monté en aval d'un traitement amont et/ou monté en amont d'un traitement aval.

13. Procédé de diafiltration selon l'une des revendications 1 à 12, dans lequel x va de 3 à 10.

Figur 1

**Figur 2**

**Figur 3**

EP 4 509 206 B1

Figur 4

EP 4 509 206 B1

**Figur 5A**

**Figur 5B**

**Figur 5C**

EP 4 509 206 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3441249 C
- US 2005285100 A
- DE 10022259 A1